## Europäisches Patentamt
## European Patent Office
## Office européen des brevets

(19)

(11) Veröffentlichungsnummer: **0 176 863**
**B1**

(12) **EUROPÄISCHE PATENTSCHRIFT**

(45) Veröffentlichungstag der Patentschrift:
14.06.89

(51) Int. Cl.⁴: **A 61 B 5/12, G 06 F 15/42**

(21) Anmeldenummer: 85111766.3

(22) Anmeldetag: 17.09.85

(54) Gehörmessgerät.

(30) Priorität: 02.10.84 DE 3436127

(43) Veröffentlichungstag der Anmeldung:
09.04.86 Patentblatt 86/15

(45) Bekanntmachung des Hinweises auf die Patenterteilung:
14.06.89 Patentblatt 89/24

(84) Benannte Vertragsstaaten:
AT DE GB IT

(56) Entgegenhaltungen:
DE-A- 3 108 407
FR-A- 2 398 364
US-A- 3 808 354
US-A- 4 284 847
US-A- 4 356 825

HEWLETT-PACKARD JOURNAL, Band 31, Nr. 11,
November 1980, Seiten 19-23, Palo Alto, CA, US; J.M.
GOLDBERG: "Self-test and serviceability for
dependable central patient monitoring"

(73) Patentinhaber: **Siemens Aktiengesellschaft Berlin und München, Wittelsbacherplatz 2, D-8000 München 2 (DE)**

(72) Erfinder: **Hohn, Werner Henri, Dr., Boggasse 1, D-8525 Uttenreuth (DE)**

## Beschreibung

Die Erfindung betrifft eine Anschlusseinheit für einen an ein Audiometer zur Einstellung von Hörgeräten anschliessbaren Rechner nach dem Oberbegriff des Patentanspruchs 1.

Wenn ein Audiometer zur Bestimmung der Hörfähigkeit von Personen oder zur Auswahl und Feststellung der günstigsten Einstellung eines Hörgerätes von einem Rechner automatisch gesteuert werden soll, müssen alle Bedienungsvorgänge, wie z.B. Wahl von Lautstärke oder Frequenzgang, elektrisch fernsteuerbar sein. Ausserdem muss dafür gesorgt werden, dass die in grosser Anzahl erforderlichen Steuerleitungen von wenigen Rechnersteuerleitungen nacheinander bedient werden können und dass die gewählten Funktionen bis zum Widerruf eingestellt bleiben. Diese Aufgaben werden bekanntlich von Rechnerinterfaces wahrgenommen.

Die bekannten Anschlusseinheiten, die z.B. nach den Vorschriften IEEE 488 oder V 24 arbeiten, sind wegen der in bestimmter zeitlicher Folge erforderlichen Signale zur Steuerung des Datentransfers zu aufwendig, um jede Baugruppe eines Praxisgerätes damit ausrüsten zu können. Sie erfordern überdies Signalfolgen, die einfachste, für die Audiometrie oder Hörgeräteanpassung an sich ausreichende Rechner ohne Zusatzbausteine nicht liefern können.

Aus der DE-A 3 108 407 ist ein Tonaudiometer bekannt, das mit Mitteln zum knackfreien Schalten oder Tasten der von einem Tonfrequenzgenerator gelieferten Tonfrequenzsignale ausgerüstet ist. Das Tonaudiometer umfasst einen ersten elektronischen Schalter, der mit einem Tonfrequenzgenerator in Reihe liegt. Ein zweiter elektronischer Schalter liegt im Gegenkopplungsweg eines Tonfrequenzverstärkers. Gegebenenfalls kann noch ein dritter elektronischer Schalter vorgesehen sein, der den Eingangswiderstand des Tonfrequenzverstärkers überbrückt. Die elektronischen Schalter werden über Steuerleitungen von einer gemeinsamen Steuerspannungsquelle gesteuert. Die Steuerspannungsquelle liefert wahlweise ein positives Potential, ein Bezugspotential oder eine Impulsfolge. Die elektronischen Schalter sind Optokoppler, die zum knackfreien Schalten, jedoch nicht zum Trennen eines Adress- oder Datenbusses verwendet werden.

Eine Gehörmesseinrichtung, die über eine Anschlusseinheit mit einem Rechner verbunden und bei der der Rechner galvanisch von der Anschlusseinheit getrennt ist, wird in der US-A 4 284 847 beschrieben. Die bekannte Einrichtung ist sehr aufwendig, da ein 16 bit Adressenbus und ein 8 bit Datenbus benötigt werden.

Um ein Low Cost-Gerät für Handbetrieb aufzubauen, das auf einfache Weise durch einen Rechner zur automatischen Steuerung erweitert werden kann, ist es erforderlich, dass die zu steuernden Baugruppen des Audiometers, z.B. ein Tongenerator, ein Lautstärkesteller etc., durch einfache Handschalter angesteuert werden können. Eine solche Möglichkeit ist auch bei rechnergesteuerten Geräten von grossem Vorteil, da im Fehlerfall durch Ersatz des Rechners durch einen Schalterbaustein festgestellt werden kann, welche Fehler vorher allein durch den Rechner verursacht wurden. Für diese Aufgabe eignen sich die bisher üblichen Anschlusseinheiten weniger gut, da sie zu kompliziert arbeiten.

Audiometer werden im medizinischen Bereich mit erhöhten Anforderungen an die elektrische Sicherheit eingesetzt. Üblicherweise wird der Anschluss der Audiometer an fremde Rechnerkomponenten, z.B. Zentralrechner eines Institutes, die nur geringeren Sicherheitsanforderungen genügen, verlangt; deshalb muss eine galvanische Trennung für die höhere geforderte Differenzspannung entweder zwischen dem Rechner und den zu steuernden Komponenten oder zwischen dem Rechner und den fremden Rechnerkomponenten vorgesehen werden. Die erste Lösung ist günstiger, da dabei die Störung der zeitweise extrem geringen Nutzsignale des Audiometers durch die galvanische Verkopplung mit den Steuersignalen des Rechners entfällt.

Die preiswerten Koppelelemente, wie z.B. Optokoppler für Differenzspannungen über 4 kV, ergeben bei der für schnelle Steuervorgänge notwendigen Parallelübertragung Zeitdifferenzen der Steuersignale. Die bisher bekannten Anschlusseinheiten erfordern dabei zur sicheren Funktion zusätzlichen Aufwand, z.B. in Form von Laufzeitausgleichsschaltungen.

Der Erfindung liegt die Aufgabe zugrunde, eine kostengünstige Anschlusseinheit der eingangs genannten Art anzugeben, die an einen Rechner und an die Software extrem geringe Anforderungen stellt und eine galvanische Trennung mit Hilfe von möglichst wenigen, extrem spannungsfesten Koppelelementen ermöglicht.

Erfindungsgemäss wird diese Aufgabe durch die im Patentanspruch 1 gekennzeichneten Merkmale gelöst. Vorteilhafte Ausgestaltungen der Erfindung sind in den Patentansprüchen 2 bis 10 gekennzeichnet.

Die Erfindung geht davon aus, dass folgende Punkte erfüllt sind:

1. Einzelne Baugruppen des Audiometers sollen beliebig kombinierbar sein und extrem störsicher gesteuert werden.

2. Der Steuervorgang darf die Signale für die Gehörprüfung nicht stören, deren Spannungen im $\mu$V-Bereich liegen können.

3. Die Baugruppen des Audiometers sollen dem Rechner auf Abruf unabhängig von den Steuerleitungen für Programmverzweigungen ihre Anwesenheit durch Antwortsignale melden sowie Messergebnisse übermitteln können.

4. Die Anschlusseinheit soll so aufgebaut sein, dass die Baugruppen des Audiometers über mehrere Schalter durch die Anschlusseinheit, jedoch auch ohne Rechner, von Hand bedient werden können.

5. Zur Unterdrückung von Umschaltstörungen, z.B. Geräuschen, soll die Hardware selbsttätig ein Austastsignal erzeugen.

Eine erfindungsgemässe Anschlusseinheit erfüllt die vorstehenden Punkte, wenn für die Ansteuerung des Audiometers mindestens zwei Signalleitungen, wegen der günstigen, im Handel vorhandenen Angebote vorzugsweise acht Signalleitungen, und für die Antwortsignale vorzugsweise eine Signalleitung verwendet werden, wenn die Adressworte der einzelnen Audiometer-Baugruppen und die Datenworte zeitlich nacheinander gesendet werden und dabei jedes auf ein Adresswort folgende Datenwort nach einer vorwählbaren Wartezeit ohne ein vom Rechner gesendetes Taktsignal in einem dieser Adresse zugeordneten Datenspeicher gespeichert wird. Dadurch, dass die Ansteuerungs- und die Antwortsignale über getrennte Leitungen geführt werden, entfallen relativ aufwendige Umschaltbausteine bei jeder Baugruppe. Durch das zeitliche Verschachteln verringern sich die Kosten für die galvanische Entkopplung sowie die Verbindung jeder Audiometer-Baugruppe mit dem Rechner durch Handverdrahtung oder eine Motherboardplatine.

Damit Störimpulse auf dem Datenbus, die beim Schalten entstehen und die eine falsche Adresse ergeben können, unwirksam werden, wird die Wartezeit zwischen dem Abschalten des Adresswortes und der Übernahme des folgenden Datenwortes durch einen Integrator für das Ausgangssignal des jeweiligen Adressdecoders bewirkt.

Zur Erfüllung der genannten Anforderungen ist weiterhin ein Teil der durch die Anzahl der Steuerleitungen maximal möglichen Datenworte für Adressen reserviert und der Adressendecoder so aufgebaut, dass er selbsttätig ein Steuersignal wählbarer Dauer erzeugt, das während der Befehlsausführung Störspannungen austasten kann, die durch die Umschaltungen der Nutzsignalwege entstehen, indem z.B. der Hörerausgang mit einem «weich» schaltenden Optokoppler kurzzeitig abgeschaltet wird.

Ausserdem wird ein Antwortsignal erzeugt, das z.B. eine Leuchtanzeige einschalten kann, um anzuzeigen, dass der zu einer Adresse gehörende Befehlsspeicher für die Übernahme der neuen Daten vorbereitet ist, oder das dem Rechner mitteilt, ob eine Programmverzweigung möglich ist, welche die gerade adressierte Funktion erfordern würde.

Um die Anzahl der Leitungen möglichst gering zu halten, kann der Signalfluss eines wählbaren Anteils der Steuerleitungen umgekehrt werden, die Umkehrung kann durch einen Rechnerbefehl oder automatisch nach Ablauf eines vom zugehörigen Adressdecoder gestarteten Zeitgliedes für eine wählbare Zeitspanne bewirkt werden; dadurch ist eine schnelle Parallelübertragung von Messwerten, z.B. der Reaktionszeit oder der Antwortdauer eines Probanden auf einen Reiz, vom Audiometer zum Rechner für die Auswertung möglich.

Die für ein Audiometer ausreichend leistungsfähigen Mikrocomputer verfügen im allgemeinen über einen Datenausgang mit acht Leitungen, der auch als Eingang umgeschaltet werden kann, und häufig über einen zusätzlichen Dateneingang mit mindestens einer Leitung. Damit das Audiometer von unterschiedlichen Rechnern gesteuert werden kann, werden vorzugsweise acht Steuerleitungen vorgesehen und die Antwortsignale aller Adressdecoder mit Hilfe von Oder-Schaltungen über eine einzige Antwortleitung gesendet.

Die Anwortsignale zur Identifizierung von Audiometer-Baugruppen mit gleicher Adresse, wie z.B. ein Tongenerator und ein Mitlauffilter, können nach dem Abschalten ihrer Adresse verschieden verzögert gesendet werden, so dass auch in diesem Fall die Anwesenheit aller für eine Programmverzweigung erforderlichen Baugruppen durch Zählen der Antworten geprüft werden kann.

Zur Abfrage von Messwerten, die in Form eines Bitmusters im Audiometer vorliegen, wird für jedes Bit ein eigener Adressdecoder vorgesehen, dessen Antwortsignal vom Zustand des zugeordneten Bits beeinflusst wird, d.h., dass z.B. nur dann ein Antwortsignal gegeben wird, wenn das zugeordnete Bit gesetzt ist. Durch serielle Anwahl dieser Adressdecoder kann der Rechner das Bitmuster abfragen.

Sind mehr als zwei Adressdecoder erforderlich, so ist es günstig, wenn eine erste Adresse und ein erstes Datenwort eine Unteradresse definieren, unter der mit Hilfe einer zweiten Adresse ein zweites Datenwort gespeichert wird, weil dann die Gesamtzahl der Bauteile für die Befehlsspeicherung geringer ist als bei je einem Adressdecoder pro Befehlsspeicher. Auf diese Weise können natürlich auch mehrere Bauteile zusammengefasst werden.

Zum Erreichen hoher Störsicherheit können alle Steuer- und Antwortleitungen etwa mittels Optokopplern für Differenzspannungen grösser als 4 kV galvanisch vom Rechner getrennt werden. Die Audiometer-Baugruppen können dann mit frei wählbaren Steuerspannungen betrieben werden, die erheblich von denen des Rechners abweichen können. Sie können z.B. mit Spannungen zwischen $U_{Low} = -7,5$ V und $U_{High} = +7,5$ V betrieben werden, um mit Standard-CMOS-Bauteilen Wechselspannungen ohne Vorspannung und ohne Pegelshifter zu schalten.

Zum Erreichen hoher Störsicherheit können alle Befehle zunächst zwischengespeichert werden und bei Wiederholung der Adresse und des Datenwortes nur bei Übereinstimmung endgültig gespeichert werden. Dies bewirkt, dass ein Störimpuls, der eine falsche Adresse vortäuscht, nicht zu einer eventuell für den Probanden gefährlichen Einstellung des Audiometers, z.B. wegen zu grosser Lautstärke, führen kann.

Zum Aufbau eines einfachen, handbetriebenen Audiometers können eine Adresse und ein Datenwort mit je einem mehrpoligen Schalter vorgewählt werden. Mit einem mehrpoligen Umschalter kann dann zunächst die erste Adresse, dann das zugehörige Datenwort, dann nach Ändern des Adressschalters die zweite Adresse und nach Ändern des Datenwortschalters das zweite Datenwort auf den Datenbus geschaltet werden. Der jeweilige Zustand der Befehlsspeicher kann dabei zur Kontrolle angezeigt werden, z.B. durch Leuchtdioden oder Flüssigkristallanzeigen.

Wenn für jede Adresse ein eigener Befehlwahlschalter vorgesehen wird, kann die jeweilige Adresse fest verdrahtet werden und mit einem mehrpoligen Stufenschalter oder -taster mit mindestens drei Stellungen vom Aus-Zustand über die Adresse zum zugehörigen Befehl geschaltet werden. Es muss dafür gesorgt werden, dass vor dem Wiedererreichen des Aus-Zustandes die Adresse nicht ohne Befehl wiederholt wird. Dadurch ergibt sich der Vorteil, dass der Benutzer ein derartiges Audiometer in der gleichen Weise bedienen kann, wie er es von den bisherigen handbedienten Geräten gewohnt ist.

Durch die Anwendung vorliegender Erfindung könnten z.B. das computergesteuerte Audiometer nach der US-PS 3 808 354, der audiometrische Rechner nach der US-PS 3 970 785 und das Audiometer nach der CA-PS 950 106 dahingehend verbessert werden, dass durch Ausrüstung aller Bauteile, wie z.B. Oszillator oder Rauschgenerator, mit Adressdecodern und Befehlsspeichern für z.B. neun Signalleitungen auch einfachste Personalcomputer ohne Änderung angeschlossen werden könnten. Dabei stehen Signale zur Störspannungsaustastung ohne zusätzlichen Aufwand zur Verfügung.

Für alle rechnergesteuerten Audiometer gilt, dass sich durch Einsatz von Trennelementen, z.B. von Optokopplern, in der oben beschriebenen Art zwischen dem Rechner und dem Audiometer die erhöhten Sicherheitsvorschriften kostengünstiger erfüllen lassen als durch geschirmte Ausgangstransformatoren, besondere Isolationen der vom Probanden benutzten Teile, wie z.B. den Kopfhörern, oder durch spezielle Netztrenntransformatoren für den Rechner und seine Peripherie und eine zusätzliche Schutztrennstelle in der Verbindung zu externen Datenverarbeitungsanlagen.

Weiter ist es auf einfache Weise möglich, statt eines Rechners ein Handbedienpult anzuschliessen, falls der Benutzer Kosten sparen oder vorerst mit mechanisch zu bedienenden Audiometern in gewohnter Weise Gehörprüfungen durchführen möchte.

Audiometer mit vielen Hörern für die gleichzeitige Prüfung mehrerer Personen z.B. nach US-PS 3 808 354 oder GB-PS 1 599 367 könnten durch die Erfindung die Verbesserung erhalten, dass der Rechner die Anzahl der angeschlossenen, jeweils einem Probanden zugeordneten Audiometer-Baugruppen oder die Anwesenheit von Audiometer-Baugruppen für Spezialtests automatisch erkennt und entsprechende Programmverzweigungen durchführt.

Bei vielen Audiometern, insbesondere aber bei vollautomatisch arbeitenden Audiometern, wie z.B. nach CA-PS 950 106, kann duch die erfindungsgemässe Kontrolle der Ausgangssignale und Meldung von Fehlern an den Rechner der Vorteil erreicht werden, dass der Proband automatisch aufgefordert wird, einen anderen Kopfhörer zu benutzen.

Weitere Einzelheiten und Vorteile der Erfindung werden nachfolgend anhand der in den Figuren dargestellten Ausführungsbeispiele weiter erläutert.

In der Fig. 1 ist in einem schematischen Übersichtsblockschaltbild eine erfindungsgemässe Anschluseinheit eines Rechners an ein elektronisch steuerbares Audiometer dargestellt,

in der Fig. 2 ein Prinzipblockschaltbild der am Eingang der Anordnung nach Fig. 1 liegenden Decoder,

in der Fig. 3 eine Anordnung zur Übertragung von Messergebnissen vom Audiometer zum Rechner,

in der Fig. 4 ein vereinfachter Aufbau der Decoder-Speicher-Kombination,

in der Fig. 5 eine Sicherungsvorrichtung, die in der Übertragung der Daten für die Steuerung der Baugruppen des Audiometers gegen Fehlsteuerung eingesetzt werden kann,

in der Fig. 6 ein mehrpoliger Schalter zum manuellen Betrieb des Audiometers ohne Rechner und

in der Fig. 7 eine Ausbildung des mehrpoligen Schalters nach Fig. 6.

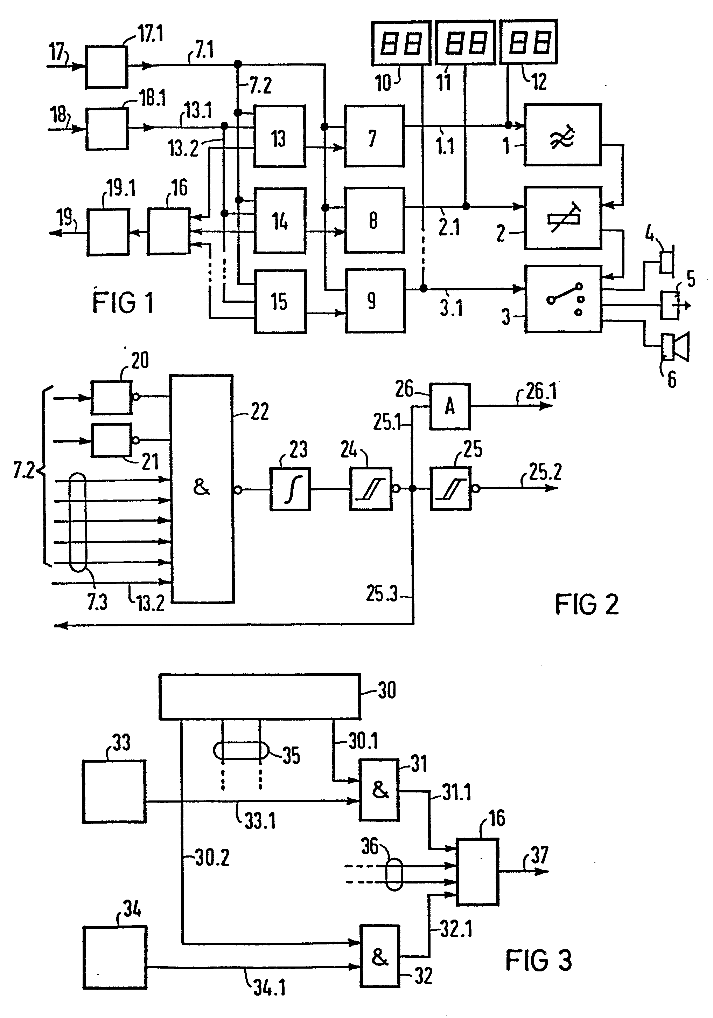

In der Fig. 1 ist mit 1 ein Tongenerator bezeichnet, der über einen Pegelsteller 2 und einen Wahlschalter 3 zur Ansteuerung mit einem an das Ohr anlegbaren Hörer 4, einem akustische Schwingungen auf die Haut übertragenden Vibrator 5 oder einem Lautsprecher 6 verbunden ist, um den Prüfschall auf eine zu untersuchende Person zu übertragen. Die jeweilige Funktion der vom Tongenerator 1, dem Pegelsteller 2 und dem Wahlschalter 3 gebildeten Baugruppen des Audiometers wird mittels Datenspeicher 7 bis 9 bestimmt. Die momentane Einstellung ist dann an alphanumerischen Anzeigetafeln 10 bis 12 ersichtlich.

Zur Einstellung des Audiometers werden von einem Rechner, der nicht gesondert dargestellt ist, Signale übertragen, die von Adressdecodern 13 bis 15 in die Datenspeicher 7 bis 9 gelangen. Dabei ist zur Vermeidung von Störungen vorausgesetzt, dass die zugeordnete Adresse nach einer Mindestverweilzeit, die länger als zu erwartende Störungen ist, abgeschaltet wird.

Während der Zeit, während der eine dem jeweils anzusprechenden Adressdecoder 13 bis 15 zugeordnete Adresse anliegt, wird über ein ODER-Gatter 16 und einen Optokoppler 19.1 ein von einem der Adressdecoder 13 bis 15 kommendes Antwortsignal zum Rechner gesendet.

Um eine galvanische Trennung des Rechners vom Audiometer zu erhalten, sind in Steuerleitungen 17 und 18 Optokoppler 17.1 und 18.1 vorgesehen. Dabei kann die Steuerleitung 17 ebenso wie eine weiterführende Leitung 7.1 zur Datenübertragung siebenfach ausgelegt sein. Auch eine Leitung 1.1 zwischen dem Datenspeicher 7 und dem Tongenerator 1 sowie eine Leitung 2.1 zwischen dem Datenspeicher 8 und dem Pegelsteller 2 sowie eine Leitung 3.1 zwischen dem Datenspeicher 9 und dem Wahlschalter 3 können in Anpassung an die Bedürfnisse der Steuerung der Baugruppen des Audiometers siebenfach ausgelegt sein. Die Anzahl der Adressdecoder 13 bis 15 und der Datenspeicher 7 bis 9 lässt sich bei der siebenfachen Auslegung entsprechend der Steuerleitung 17 auf 128 erhöhen.

In der Fig. 2 sind mit 20 und 21 Blöcke bezeichnet, in welchen vom Rechner kommende Signale invertiert werden. Sie gelangen dann ebenso wie diejenigen der Leitung 7.3 zu einem NAND-Gatter 22, wo ihre Auswertung erfolgt. Das Ausgangssignal dieses NAND-Gatters gelangt dann zur Beseitigung der Wirkung von Störimpulsen auf einen Integrator 23 und über Schmitt-Trigger-Inverter 24 und 25, zwischen denen eine Leitung 25.1 abzweigt, die zu einem monostabilen Multivibrator 26 führt, der für eine bestimmte Zeit, in der Grössenordnung von 20 ms bis 0,2 sec, ein Steuersignal zur Störungsaustastung liefert. Eine Leitung 26.1 führt zu einem Schalter, der das Ausgangssignal des Pegelstellers 2 abschaltet. Eine Leitung 25.2 führt zum zugehörigen Datenspeicher 7 bis 9. Eine zwischen den Schmitt-Trigger-Invertern 24 und 25 abzweigende Leitung 25.3 führt das Antwortsignal zum ODER-Gatter 16.

Fig. 3 zeigt einen Speicher 30 für Messwerte, die vom Audiometer selbst ermittelt werden. Eine solche Signalaufnahme kann z.B. die Zeitdauer sein zwischen dem Beginn eines Reizes und dem zugeordneten Druck auf eine Antworttaste durch den Probanden. An den Speicher 30 ist über eine Leitung 30.1 ein UND-Gatter 31 angeschlossen, dessen zweitem Eingang das Antwortsignal eines Decoders 33 über die Leitung 33.1 zugeführt wird. Dadurch wird dem ODER-Gatter 16 nur dann ein Antwortsignal über die Leitung 31.1 zugeführt, wenn ausser dem Anliegen der dem Decoder 33 zugeordneten Adresse auch der die Leitung 30.1 speisende Speicher für ein Bit des Speichers 30 gesetzt ist. Im anderen Fall erhält der Rechner kein Antwortsignal, wenn er die Adresse des Decoders 33 sendet.

Entsprechend wird das Antwortsignal eines Decoders 34 nur dann an das ODER-Gatter 16 weitergeleitet, wenn die Ausgangsleitung 30.2 eines anderen Speichers High-Pegel aufweist.

Wie durch gestrichelte Leitungen 35 in der Fig. 3 angedeutet, können weitere Speicher des zum Rechner zu übertragenden Bitmusters aufnehmenden Speichers 30 mit Hilfe von nicht gesondert dargestellten weiteren UND-Gattern die Weiterleitung der Antwortsignale weiterer, in der Zeichnung ebenfalls weggelassener Decoder zum ODER-Gatter 16 über eine der Leitungen 36 beeinflussen. Durch serielle Anwahl aller Adressdecoder, die dem Speicher 30 zugeordnet sind, kann der Rechner das Bitmuster abfragen. Die Leitung 37 führt über den Koppler 19.1 zum Rechner.

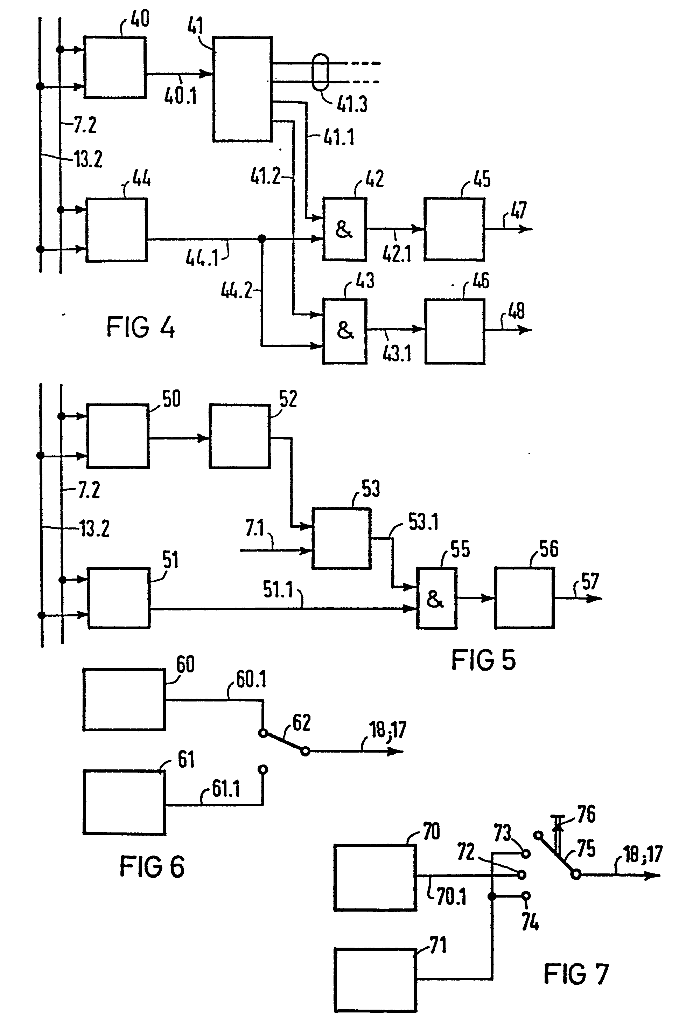

In der Fig. 4 ist eine Anordnung dargestellt, bei welcher eine erste Adresse und ein erstes Datenwort eine Unteradresse definieren, unter der mit Hilfe einer zweiten Adresse ein zweites Datenwort gespeichert wird. Dies erfolgt, indem durch Signale vom Rechner über die Leitungen 7.2 und 13.2 von einem Decoder 40 über eine Leitung 40.1 auf einen Speicher 41 ein Speicherungsbefehl übertragen wird. Von diesem Speicher 41 aus kann über Leitungen 41.1 und 41.2 sowie UND-Gatter 42 und 43 die Weiterleitung des Speicherungsbefehls eines Decoders 44, der ebenfalls an den Leitungen 7.2 und 13.2 liegt, an die Befehlsspeicher 45 und 46 gesteuert werden. Die gestrichelten Leitungen 41.3 deuten die mögliche Ergänzung durch zusätzliche weitere UND-Gatter und Speicher an. Die Weitergabe der gespeicherten Steuersignale an Audiometer-Baugruppen, wie z.B. den Tongenerator 1 oder Pegelsteller 2 etc., erfolgt über Leitungen 47 und 48.

Mit der Anordnung nach Fig. 5 wird hohe Störsicherheit erreicht, indem alle für den Speicher 56 bestimmten Befehle zunächst im Speicher 52 zwischengespeichert werden und bei Wiederholung der Adresse und des Datenwortes nur nach Übereinstimmung endgültig gespeichert werden. Dazu sind über die Leitungen 7.2 und 13.2 mit dem Rechner verbundene Decoder 50 und 51 vorgesehen, die ihre Signale vom Rechner erhalten. Vom Decoder 50 gelangt ein Speicherungsbefehl auf einen Speicher 52 und von diesem auf einen Komparator 53, in den auch sieben Leitungen 7.1 gelangen und das jeweils gültige Datenwort übertragen.

Ausserdem ist der Komparator 53 über eine Leitung 53.1 mit einem UND-Gatter 55 verbunden, zu dem auch vom Decoder 51 eine Leitung 51.1 führt. Dadurch erhält der Speicher 56 nur dann einen Speicherungsbefehl, wenn der Komparator 53 Übereinstimmung des gespeicherten mit dem zur Zeit am Bus anliegenden Datenwort meldet und der Decoder 51 einen Speicherungsbefehl erzeugt. Über eine Leitung 57 können Steuersignale an die Audiometer-Baugruppe abgegeben werden.

In der Fig. 6 ist ein mehrpoliger Umschalter 62 gezeichnet, der anstelle eines Rechners Bit-Muster an die Leitungen 17 bis 18 der Fig. 1 schalten kann. Durch den achtpoligen Schalter (zwei Hexadezimalschalter) 60 kann ein Adresswort und durch den siebenpoligen Schalter 61 ein Datenwort vorgewählt werden und durch den Schalter 62 können diese Worte abwechselnd auf den aus den Leitungen 17 und 18 bestehenden Daten- und Adressbus gegeben werden, wobei eine Befehlsgabe stets durch ein Datenwort und nicht durch eine Adresse abgeschlossen wird. Dabei wird die Leitung 18 nur vom Adresswahlschalter 60 gespeist.

In Fig. 7 sind ein fest verdrahteter Adresscoder 70, ein Datenwort-Schalter 71 und ein mehrpoliger Umschalter 75 dargestellt, von denen je ein Exemplar für jede zu steuernde Baugruppe eines Audiometers vorhanden ist, um statt mit Rechner- mit Handsteuerung arbeiten zu können. Durch den Schaltarm 75, der, wie durch einen Pfeil 76 angedeutet, durch eine Feder in die Aus-Stellung gezogen wird, kann die Kontaktierung so erfolgen, dass vom Aus-Zustand über den an den Schaltpunkten 73 anliegenden Befehl und die an den Schaltpunkten 72 anliegende Adresse wieder zum ebenfalls an den Schaltpunkten 74 anliegenden Befehl und zurück zum Aus-Zustand geschaltet werden kann, wobei eine Befehlsgabe stets mit einem Befehl und nicht mit einer Adresse abgeschlossen wird. Durch eine mechanische, elektrische oder elektronische Verriegelung wird dabei verhindert, dass Fehler durch gleichzeitiges Bedienen mehrerer derartiger Schalter verursacht werden können. Der Schaltarm 75 ist mit den Leitungen 17, 18 der Fig. 1 verbunden. Dabei wird die Leitung 18 nur vom Adresscoder 70 gespeist.

## Patentansprüche

1. Anschlusseinheit für einen an ein Audiometer zur Einstellung von Hörgeräten anschliessbaren Rechner, die eine Steuerung von Audiometer-Baugruppen (Tongenerator, Pegelsteller u.dgl.) durch den Rechner ermöglicht, wobei die Anschlusseinheit galvanisch vom Rechner getrennt und mit Decodern und Speichern verbunden ist, von denen Steuerleitungen ausgehen, dadurch gekennzeichnet, dass die Steuerleitungen (17 bis 19) der Anschlusseinheit zum Rechner galvanisch trennende Koppler (17.1 bis 19.1) enthalten und andererseits mit Adressdecodern (13 bis 15) verbunden sind, die ihrerseits an Datenspeichern (7 bis 9) liegen, von denen aus Steuerleitungen zu den Audiometer-Baugruppen (1 bis 3) führen, dass Antwortleitungen zwischen den Adressdecodern (13 bis 15) und dem in einer dieser Steuerleitungen liegenden galvanischen Koppler (19.1) in einem ODER-Gatter (16) zusammengeführt sind, dass eine vom Rechner kommende Signalleitung (7.2) über Inverter und eine weitere vom Rechner kommende Leitung (13.2) ohne Inverter zu einem NAND-Gatter (22) geführt sind und das Ausgangssignal des NAND-Gatters über einen Integrator (23) und zwei Schmitt-Trigger (24, 25) zum zugehörigen Datenspeicher (7 bis 9) gelangt, dass zwischen den Schmitt-Triggern (24, 25) eine Abzweigung des Antwortsignals zum ODER-Gatter (16) geführt ist, derart, dass jedes auf ein Adresswort folgende Datenwort nach einer vorwählbaren Wartezeit ohne ein vom Rechner gesendetes Taktsignal in einem dieser Adresse zugeordneten Datenspeicher speicherbar ist, dass der Integrator (23) für das Ausgangssignal des Adressdecoders (13 bis 15) die Wartezeit bewirkt, dass ein Teil der durch die Anzahl der Steuerleitungen maximal möglichen Datenworte für Adressen reserviert ist, der Adressdecoder so aufgebaut ist, dass er selbsttätig ein Steuersignal zur Störspannungsaustastung während der Befehlsausführung erzeugt, dessen Zeitdauer wählbar ist, sowie ein Antwortsignal, das eine Leuchtanzeige einschalten kann, während der zu einer Adresse gehörende Befehlsspeicher für die Übernahme der neuen Daten vorbereitet ist bzw. eine Programmverzweigung veranlassen kann.

2. Anschlusseinheit nach Anspruch 1, dadurch gekennzeichnet, dass zwischen den Schmitt-Triggern (24, 25) ein monostabiler Multivibrator angeschlossen ist, der für eine bestimmte Zeit, in der Grössenordnung von 20 ms bis 0,2 sec, ein Steuersignal zur Störungsaustastung liefert.

3. Anschlusseinheit nach Anspruch 1, dadurch gekennzeichnet, dass an einer Signalleitung ein Speicher (30) liegt, der über UND-Gatter (31 und 32) mit dem ODER-Gatter (16) verbunden ist und dass die zweiten Eingänge der UND-Gatter (31, 32) von den Antwortsignalen der Decoder (33, 34) gespeist werden.

4. Anschlusseinheit nach Anspruch 1, dadurch gekennzeichnet, dass der Signalfluss eines wählbaren Anteils der Steuerleitungen (17, 18) durch einen Rechnerbefehl oder automatisch nach Ablauf eines vom zugehörigen Adressdecoder gestarteten Zeitgliedes für eine wählbare Zeitspanne umkehrbar ist.

5. Anschlusseinheit nach den Ansprüchen 1 bis 4, dadurch gekennzeichnet, dass das Audiometer über acht Steuerleitungen vom Rechner steuerbar ist und die Antwortsignale mehrerer Adressdecoder dem steuernden Rechner mit Hilfe von ODER-Schaltungen (16) über eine Antwortleitung (19) zuführbar sind.

6. Anschlusseinheit nach Anspruch 5, dadurch gekennzeichnet, dass zur Abfrage von Bit-Mustern für jedes Bit ein Adressdecoder (33, 34) vorgesehen und dessen Antwortsignal vom Zustand des zugeordneten Bits beeinflussbar ist.

7. Anschlusseinheit nach Anspruch 1, dadurch gekennzeichnet, dass zum Erreichen hoher Störsicherheit alle Steuer- und Antwortleitungen galvanisch vom Rechner getrennt sind und die Audiometer-Baugruppen mit frei wählbaren, erheblich von denen des Rechners abweichenden Steuerspannungen betreibbar sind.

8. Anschlusseinheit nach Anspruch 1 und 7, dadurch gekennzeichnet, dass zur galvanischen Trennung Optokoppler (17.1 bis 19.1) für Differenzspannungen grösser als 4 kV eingesetzt werden.

9. Anschlusseinheit nach Anspruch 1, dadurch gekennzeichnet, dass eine Adresse und ein Datenwort mit je einem mehrpoligen Schalter vorwählbar und mit einem mehrpoligen Umschalter abwechselnd auf den Bus schaltbar ist und dass zusätzlich der jeweilige Zustand der Befehlsspeicher durch Leuchtdioden oder Flüssigkristallanzeigen (10 bis 12) angezeigt wird.

10. Anschlusseinheit nach Anspruch 1, dadurch gekennzeichnet, dass für jede Adresse das Adresswort fest verdrahtet ist und ein Datenwort mit einem mehrpoligen Schalter (72 bis 75) vorwählbar ist, dass pro Adresse mit einem z.B. durch einen mechanischen Taster gesteuerten, mehrpoligen, elektronischen Stufenschalter mit vier Stellungen vom Aus-Zustand über den zugehörigen Befehl und die Adresse wieder zum zugehörigen Befehl und zurück zum Aus-Zustand geschaltet werden kann und dass elektrische oder mechanische Massnahmen Fehler durch gleichzeitiges Bedienen mehrerer derartiger Schalter verhindern.

## Claims

1. A connection unit for a computer which can be connected to an audiometer for adjusting hearing devices which makes the control of audiometer modules (audio oscillator, level setter and the like) possible by means of the computer, wherein the connection unit is metallically separated from the computer and is connected to decoders and stores from which control lines extend, characterised in that the control lines (17 to 19) comprise couplings (17.1 to 19.1) metallically separating the connection unit from the computer and connected at the other end to address decoders (13 to 15) which for their part adjoin data stores (7 to 9) from which control lines lead to the audiometer modules (1 to 3), that answer lines between the address coders (13 to 15) and the galvanic coupling (19.1) located in one of these control lines are joined together in an OR-gate (16), that a control line (7.2) coming from the computer by way of an invertor and a further line (13.2) coming from the computer are led without an invertor to a NAND-gate (22) and the output signal of the NAND-gate arrives at the associated data stores (7 to 9) via an integrator (23) and two Schmitt triggers (24, 25), that a branch of the answer signal is led between the Schmitt triggers (24, 25) to the OR-gate (16 ) so that each data word following an address word can be stored in a data store associated with this address after a waiting period that can be preselected without a clock signal being sent from the computer, that the integrator (23) effects the waiting period for the output signal of the address decoder (13 to 15), that a part of the maximum possible data words set by the number of control lines is reserved for the addresses, the address decoder is constructed so that it automatically produces a control signal for interference voltage blanking while the command is being executed whose duration can be selected, and an answer signal that can switch on an indicator light while the command store belonging to an address is prepared to receive new data or can effect a program branching.

2. A connection unit according to claim 1, characterised in that connected between the Schmitt triggers (24, 25) is a monostable multivibrator which for a certain period, of the order of 20 ms to 0.2 sec, supplies a control signal to the interference blanking.

3. A connection unit according to claim 1, characterised in that connected to a signal line is a store (30) which is connected via AND-gates (31 and 32) to the OR-gate (16) and that the second inputs of the AND-gates (31, 32) are fed by the answer signals from the decoder (33, 34).

4. A connection unit according to claim 1, characterised in that the signal flow of a selectable part of the control lines (17, 18) can be reversed for a selectable time by a computer command or automatically after the operation of a timing element started by the associated address decoder.

5. A connection unit according to claims 1 to 4, characterised in that the audiometer can be controlled by the computer via eight control lines and the answer signals of a plurality of address decoders can be supplied to the controlling computer by means of OR-circuits (16) via an answer line (19).

6. A connection unit according to claim 5, characterised in that in order to interrogate bit patterns an address decoder (33, 34) is provided for each bit and the answer signal can be influenced by the state of the associated bit.

7. A connection unit according to claim 1, characterised in that in order to obtain increased immunity to interference all control and answer lines are metallically separated from the computer and the audiometer modules can be operated by any desired control voltages differing considerably from those of the computer.

8. A connection unit according to claim 1 and claim 7, characterised in that for metallic separation optocouplers (17.1 to 19.1) are used for differential voltages higher than 4 kV.

9. A connection unit according to claim 1, characterised in that an address and a data word are preselectable, each by a multipolar switch, and are alternately switchable on the bus by a multipolar change-over switch, and that in addition the respective state of the command store is indicated by light-emitting diodes or liquid-crystal displays (10 to 12).

10. A connection unit according to claim 1, characterised in that the address word is permanently wired for each address and a data word can be preselected by a multipolar switch (72 to 75), that for each address witching is possible by a multipolar, electronic step switch having four settings controlled, e.g. by a mechanical control switch, from the off-state by way of the associated command and the address, again to the associated command and back to the off-state, and that electrical or mechanical measures can prevent errors by the simultaneous operation of a plurality of such switches.

## Revendications

1. Unité de raccordement prévue pour un ordinateur, pouvant être raccordé à un audiomètre pour régler des appareils de correction auditive, et qui permet une commande de modules de l'audiomètre (générateur de signaux acoustiques, régulateur de niveau et analogues) par l'ordinateur, l'unité de raccordement étant séparée galvaniquement par rapport à l'ordinateur et étant reliée à des décodeurs et des mémoires, d'où partent des lignes de commande, caractérisée par le fait que les lignes de commande (17 à 19) de l'unité de raccordement contiennent des coupleurs (17.1 à 19.1), séparés galvaniquement par rapport à l'ordinateur, et sont reliées, par ailleurs, à des décodeurs d'adresses (13 à 15), qui pour leur part sont raccordés à des mémoires de données (7 à 9), d'où partent des lignes de commande aboutissant aux modules (1 à 3) de l'audiomètre, que des lignes de réponse présentes entre les décodeurs d'adresses (13 à 15) et le coupleur galvanique (19,1), situé dans l'une de ces lignes de commande, sont raccordées en commun à une porte OU (16), qu'une ligne (7.2) de transmission de signaux, qui arrive du calculateur, est reliée par l'intermédiaire d'un inverseur à une porte NON-ET (22) et une autre ligne (13.2) arrivant du calculateur est reliée sans aucun inverseur à cette porte, et que le signal de sortie de la porte NON-ET parvient, par l'intermédiaire d'un intégrateur (23) et de deux déclencheurs de Schmitt (23, 24), à la mémoire de données associée (7 à 9), qu'entre les déclencheurs de Schmitt (24, 25), une dérivation du signal de réponse aboutit à la porte OU (16) de sorte que chaque mot de données succédant à un mot d'adresse peut être mémorisé, après un temps d'attente pouvant être présélectionné, et ce sans aucun signal de cadence émis par l'ordinateur, dans une mémoire de données, associée à cette adresse, que l'intégrateur (23) pour le signal de sortie du décodeur d'adresses (13 à 15) produit le temps d'attente, qu'une partie du nombre maximum possible, déterminé par le nombre des lignes de commande, de mots de données est réservée pour des adresses, que le décodeur d'adresses est constitué de telle sorte qu'il produit automatiquement un signal de commande pour supprimer la tension parasite pendant l'exécution d'une instruction, signal dont la durée peut être sélectionnée, ainsi qu'un signal de réponse, qui peut brancher un dispositif d'affichage à lampe, tandis que la mémoire d'instructions associée à une adresse est préparée pour la prise en charge des nouvelles données ou peut déclencher un embranchement du programme.

2. Unité de raccordement suivant la revendication 1, caractérisée par le fait qu'entre les déclencheurs de Schmitt (24, 25) est branché un multivibrateur monostable, qui, pendant un intervalle de temps déterminé, de l'ordre de 20 ms à 0,2 s, délivre un signal de commande pour supprimer les parasites.

3. Unité de raccordement suivant la revendication 1, caractérisée par le fait qu'à une ligne de transmission de signaux est raccordée une mémoire (30), qui est reliée, par l'intermédiaire de portes ET (31 et 32), à la porte OU (16), et que les secondes entrées des portes ET (31, 32) sont alimentées par les signaux de réponse des décodeurs (33, 34).

4. Unité de raccordement suivant la revendication 1, caractérisée par le fait que le flux de transmission de signaux d'une partie, pouvant être sélectionnée, des lignes de commande (17, 18) peut être inversé, pendant un intervalle de temps pouvant être sélectionné, au moyen d'une instruction de l'ordinateur ou automatiquement après l'écoulement d'un retard produit par une minuterie déclenchée par le décodeur d'adresses associé.

5. Unité de raccordement suivant les revendications 1 à 4, caractérisée par le fait que l'audiomètre peut être commandé par l'ordinateur par l'intermédiaire de huit lignes de commande et que les signaux de réponse de plusieurs décodeurs d'adresses peuvent être envoyés à l'ordinateur effectuant la commande, à l'aide de circuits OU (16), par l'intermédiaire d'une ligne de réponse (19).

6. Unité de raccordement suivant la revendication 5, caractérisée par le fait qu'un décodeur d'adresses (33, 34) est prévu, pour chaque bit, pour l'interrogation de profils binaires et que le signal de réponse de ce décodeur peut être influencé par l'état du bit associé.

7. Unité de raccordement suivant la revendication 1, caractérisée par le fait que pour l'obtention d'une sécurité supérieure vis-à-vis des parasites, toutes les lignes de commande et toutes les lignes de réponse sont séparées galvaniquement par rapport à l'ordinateur, et que les modules de l'audiomètre peuvent fonctionner en étant alimentés par des tensions de commande pouvant être choisies librement et s'écartant fortement de celles de l'ordinateur.

8. Unité de raccordement suivant les revendications 1 et 7, caractérisée par le fait que pour réaliser la séparation galvanique, on utilise des optocoupleurs (17.1 à 19.1) pour des tensions différentielles supérieures à 4 kV.

9. Unité de raccordement suivant la revendication 1, caractérisée par le fait qu'une adresse et un mot de données peuvent être présélectionnés au moyen de commutateurs multipolaires respectifs et peuvent être transmis en alternance au bus au moyen d'un commutateur multipolaire et qu'en outre, l'état respectif de la mémoire d'instructions est affiché par des diodes à luminescence ou des dispositifs d'affichage à cristal liquide (10 à 12).

10. Unité de raccordement suivant la revendication 1, caractérisée par le fait que, pour chaque adresse, le mot d'adresse est transmis par un câblage fixe et qu'un mot de données peut être présélectionné à l'aide d'un commutateur multipolaire (72 à 75), que, pour chaque adresse, une commutation peut être réalisée depuis l'état d'arrêt en passant par l'instruction associée et l'adresse pour aboutir à nouveau à l'instruction associée et revenir à l'état d'arrêt, à l'aide d'un commutateur électronique multipolaire à gradins, commandé par un dispositif de manipulation mécanique et comportant quatre positions, et que des dispositions de nature électrique ou mécanique empêchent des erreurs entraînées par la commande simultanée de plusieurs commutateurs de ce type.

FIG 1

FIG 2

FIG 3

FIG 4

FIG 5

FIG 6

FIG 7